(19) Europäisches Patentamt
European Patent Office
Office européen des brevets

(11) Veröffentlichungsnummer : **0 025 083**
**B1**

(12) # EUROPÄISCHE PATENTSCHRIFT

(45) Veröffentlichungstag der Patentschrift : **03.02.82**

(21) Anmeldenummer : **80103054.5**

(22) Anmeldetag : **02.06.80**

(51) Int. Cl.³ : **C 07 D295/08**, C 07 C 91/10, C 07 C 89/02// C07C43/13, C07C93/04

(54) **Verfahren zur Herstellung von Serinol und Serinolderivaten.**

(30) Priorität : **10.09.79 CH 8182/79**

(43) Veröffentlichungstag der Anmeldung : **18.03.81 (Patentblatt 81/11)**

(45) Bekanntmachung des Hinweises auf die Patenterteilung : **03.02.82 Patentblatt 82/05**

(84) Benannte Vertragsstaaten : **AT CH DE FR GB IT LI SE**

(56) Entgegenhaltungen :
**CHEMICAL ABSTRACTS, Band 55, Nr. 19, 18. September 1961, Nr. 18575e 18576c, Columbus, Ohio, U.S.A. YUTAKA KUWADA : « XXXI. Antitumor action of 1-dialkylamino-2,3-dichloropropanes and 2-dialkylamino-1,3-dichloropropanes »**

**CHEMICAL ABSTRACTS, Band 57, Nr. 2, 23. Juli 1962, Spalte 2054d, Columbus, Ohio, US, TSUNEHIKO KUWAMURA : « Preparation and properties of higher glyceryl α,α'-diethers »**

**CHEMICAL ABSTRACTS, Band 61, Nr. 2, 20. Juli 1964, Spalte 1790h, Columbus, Ohio, US, I.D.V. IOFFE et al. : « Cholinolytic substances with alkylating groups »**

**CHEMICAL ABSTRACTS, Band 70, Nr. 23, 9. Juni 1969, Zusammenfassung Nr. 106442f, Columbus, Ohio, US, L. VELICHKOV et al. : « Preparation of bis(2-chloroethyl) amidophosphoric acid esters of morpholino-alkanols »**

(73) Patentinhaber : **EPROVA Aktiengesellschaft**
**Im Laternenacker 5**
**CH-8200 Schaffhausen (CH)**

(72) Erfinder : **Felder, Ernst, Prof. Dr.**
**Via Ceresio 49**
**CH-6826 Riva S. Vitale (CH)**
Erfinder : **Bianchi, Sergio, Dr.**
**Viale E. Caldara 11**
**I-20122 Milano (IT)**
Erfinder : **Bollinger, Heinrich, Dr.**
**Neuweg 11**
**CH-8222 Beringen (CH)**

(56) Entgegenhaltungen :
**CHEMICAL ABSTRACTS, Band 90, Nr. 19, 7. Mai 1979, Seite 553, Zusammenfassung Nr. 151509g, Columbus, Ohio, US, B. KH. KIMSANOV et al. : « Preparation of 1,3-dialkoxy-2-propanols »**

**J. AM. CHEM. SOC., Band 77, März 1955, Seiten 1 568-1 572 Washington, D.C., U.S.A. N.H. CROMWELL et al. : « β,γ-Dihalopropylamines. I. 1-Amino-2,3-dichloropropanes and 1,4-diamino-2,3-dichlorobutanes »**

**J. MED. CHEM., Band 10, Mai 1967, Seite 511, Washington, D.C., U.S.A. CH. E. WILLIAMSON et al. : « Synthesis of potential anticancer agents »**

**EP 0 025 083 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von Serinol und Serinol-Derivaten.

Gegenstand der vorliegenden Erfindung ist ein neues, vorteilhaftes Verfahren für die rationelle Herstellung von Serinol im technischen Massstab sowie von am Stickstoffatom substituierten Serinol-Derivaten.

Serinol — 1,3-Dihydroxy-isopropylamin — kann hergestellt werden durch Reduktion des Oxims von 1,3-Dihydroxyaceton. Durch Isomerisierung entsteht jedoch dabei ein kaum trennbares Gemisch von Serinol und 2,3-Dihydroxy-propylamin. Serinol wird daher im allgemeinen aus Nitromethan hergestellt. Dazu wird Nitromethan in alkalischer Lösung mit Formaldehyd zum 2-Nitro-1,3-propandiol umgesetzt ; letzteres wird katalytisch zu Serinol reduziert, wobei Nebenreaktionen auftreten können. Vergl. dazu : Schmidt et al, Ber. dtsch. chem. Ges. 52, 389 ; Langenbeck et al, Naturwissenschaften 42, (1955) 389-90 ; Schipper et al, J. Org. Chem. 26 (1961) 4145-8 ; Pfeiffer, DE-AS 27 42 981 (Schering A.G.).

Diese Methode erfordert die Handhabung des hochexplosiven Nitromethans und des noch gefährlicheren 2-Nitro-1,3-propandiols oder dessen Natriumsalz. Unfälle mit Nitroalkanen haben zu einer derartigen Verschärfung der behördlichen Vorschriften geführt, dass aliphatische Nitroverbindungen nur noch in hochabgesicherten, weitabliegenden Anlagen verwendet werden dürfen, deren Einrichtung und Betrieb nur für Grossprodukte lohnend ist.

Es erwies sich daher als schwierig, Serinol in ausreichender Menge zu kaufen oder aus Nitromethan herstellen zu lassen, da kaum jemand die hohen Risiken eingehen wollte oder durfte.

Es wurde nun gefunden, dass sich Serinol unter Umgehung der geschilderten Schwierigkeiten aus sehr billigen Rohstoffen einfach und mit hervorragender Reinheit frei von störenden Isomeren herstellen lässt, wenn man Epichlorhydrin in bekannter Weise mit einem niedrigen Alkohol in Gegenwart starker Alkalien zu 1,3-Dialkoxy-isopropanol umsetzt, dieses nach bekannten Methoden ins entsprechende 1,3-Dialkoxy-isopropyl-halogenid überführt, letzteres durch Umsetzung mit Ammoniak in das 1,3-Dialkoxy-isopropylamin umwandelt und schliesslich die Aethergruppen durch Erhitzen mit einer Halogenwasserstoffsäure HCl, HBr oder HJ spaltet. Es hat sich gezeigt, dass zu dieser Aetherspaltung bereits siedende Salzsäure ausreicht. Die Anwendung von Salzsaüre hat den zusätzlichen Vorteil, dass in siedender Salzsäure kaum Umwandlungen von HO-Funktionen in Cl-Funktionen erfolgen. Alle Syntheseoperationen lassen sich sehr leicht im technischen Massstabe und mit hervorragenden Ausbeuten durchführen.

Als niedrigen Alkohol für die Umsetzung mit Epichlorhydrin kommt in erster Linie Methanol in Betracht. Andere niedrige Alkohole wie Aethanol und Propanol sind jedoch analog anwendbar.

Wenn man zur Umwandlung des 1,3-Dialkoxy-isopropylhalogenides anstelle von Ammoniak ein primäres oder sekundäres Amin verwendet, so erhält man als Synthese-Endprodukte eine Reihe von wertvollen 1,3-Dihydroxy-isopropylaminen, die wie Serinol selbst als Zwischenprodukte für die Herstellung von pharmazeutischen Wirkstoffen, beispielsweise von Röntgenkontrastmitteln, Cytostatika oder Psychopharmaka u.a.m. sowie teilweise zur Salzbildung mit starken organischen Säuren beispielsweise 3,5-substituierten 2,4,6-Trijod-benzoesäuren geeignet sind.

Als derartige am Stickstoffatom substituierte Serinol-Derivate kommen in Betracht 1,3-Dihydroxy-isopropylamine der allgemeinen Formel (IV)

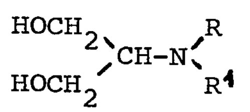

(IV)

worin R vorzugsweise Wasserstoff, Methyl, Äthyl, Hydroxyäthyl, Dihydroxypropyl oder zusammen mit $R^1$ und dem N-Atom Bestandteil eines Pyrrolidin-, Piperidin-, Morpholin-oder Piperazin-Ringes und $R^1$ Methyl, Äthyl oder Hydroxyalkyl, Di-, Tri-, Tetra- oder Pentahydroxyalkyl mit 2 bis 6 C-Atomen oder zusammen mit R und dem N-Atom einen der genannten heterocyclischen Ringe bedeuten.

Die meisten der einschlägigen Verbindungen sind in der Literatur bisher nicht beschrieben worden. Bekannt sind :

1,3-Dihydroxy-2-dimethylamino-propan
1,3-Dihydroxy-2-diäthylamino-propan
1,3-Dihydroxy-2-(2'-hydroxyäthyl)-amino-propan
1(N)-(1,3-Dihydroxyisopropyl)-piperidin und
4(N)-(1,3-Dihydroxyisopropyl)-morpholin.

1,3-Dihydroxy-2-2-dimethylaminopropan wurde nach Ioffe et al, Zhur. Obshch. Khim. *34* (4), 1336-41 [Chemical Abstracts *61* (1964) 1790h] aus 2-Nitro-1,3-propandiol durch Hydrieren in AcOH in Gegenwart von Formalin und Raney-Nickel gewonnen.

Ausbeute 42 %.

1,3-Dihydroxy-2-diäthylamino-propan wurde nach Y. Kuwada et al, Chem. Pharm. Bull. (Tokyo) *8*, 807-14 (1960) [Chemical Abstracts *55* (1961) 18575h] durch Umsetzung von Brommalonsäurediäthylester mit Diäthylamin und Reduktion des erhaltenen Diäthylaminomalonsäurediäthylesters mit Lithiumaluminiumhydrid synthetisiert.

1,3-Dihydroxy-2-(2'-hydroxyäthyl)-amino-propan wurde nach Williamson et al, J. Med. Chem. *10* (3), 511 (1967) [Chemical Abstracts *67* (1967) 64368s] aus Serinol durch Umsetzung mit Äthylenoxid erhalten.

Ferretti et al, Tetrahedron Letters *1964* (38-40), 2975-9 [Chemical Abstracts *62* (1965) 1536g] hat N-(1,3-Dihydroxyisopropyl)-morpholin aus N-Allylmorpholin durch Behandlung mit $Ag(BzO)_2J$ und Verseifung des erhaltenen Dibenzoates hergestellt.

Neu sind insbesondere die am Stickstoffatom substituierten 1,3-Dihydroxy-isopropylamine der allgemeinen Formel (III)

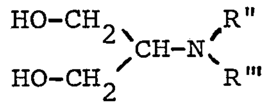
(III)

worin R" Wasserstoff, Methyl, Äthyl, Hydroxyäthyl, Dihydroxypropyl, oder zusammen mit R"' und dem N-Atom Bestandteil eines Pyrrolidin- oder Piperazin-Ringes und R"' Di-, Tri-, Tetra- oder Pentahydroxyalkyl mit 3 bis 6 C-Atomen oder zusammen mit R" und dem N-Atom einen der genannten heterocyklischen Ringe bedeuten.

Das erfindungsgemässe Verfahren zur Herstellung von Serinol und von am Stickstoffatom substituierten Serinol-Derivaten ist demnach dadurch gekennzeichnet, dass man ein aus Epichlorhydrin über 1,3-Dialkoxy-isopropanol nach bekannten Methoden leicht erhältliches niedriges 1,3-Dialkoxy-isopropylhalogenid mit Ammoniak oder einem Amin der allgemeinen Formel (I)

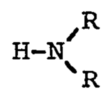
(I)

worin R vorzugsweise Wasserstoff, Methyl, Äthyl, Hydroxyäthyl, Dihydroxypropyl, oder zusammen mit R' und dem N-Atom Bestandteil eines Pyrrolidin-, Piperidin-, Morpholin- oder Piperazin-Ringes und R' Wasserstoff, Methyl, Äthyl oder Hydroxyalkyl, Di-, Tri-, Tetra- oder Pentahydroxyalkyl mit 2 bis 6 C-Atomen oder zusammen mit R und dem N-Atom einen der genannten heterocyklischen Ringe bedeuten, umsetzt, und im erhaltenen 1,3-Dialkoxy-isopropylamin bzw. 1,3-Dialkoxy-isopropylamin-Derivat der Formel (II)

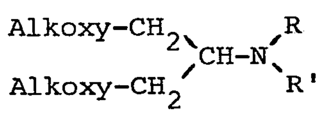
(II)

worin Alkoxy niedrige Alkoxy-Reste mit 1 bis 3 Kohlenstoff-Atomen sind und R sowie R' die oben definierte Bedeutung haben, die Äthergruppen durch Erhitzen mit einer Halogenwasserstoffsäure spaltet.

Das bevorzugte Verfahren besteht darin, dass man zur erfindungsgemässen Synthese 1,3-Dimethoxy-isopropylchlorid als Zwischenprodukt verwendet, und die Ätherspaltung durch Erhitzen mit Salzsäure bewerkstelligt.

Das einfachste technische Verfahren zur Herstellung von Serinol ist demnach dadurch gekennzeichnet, dass man 1,3-Dimethoxy-isopropylchlorid zunächst mit überschüssigem Ammoniak umsetzt, und erhaltenes 1,3-Dimethoxy-isopropylamin durch Rückflusskochen mit wässriger Salzsäure in Serinol überführt, wobei man den zur Beschleunigung der Reaktion verbrauchten Chlorwasserstoff laufend oder periodisch ersetzt.

Typische Verfahrensprodukte sind demgemäss : Serinol ( = 1,3-Dihydroxy-isopropylamin) sowie die 1,3-Dihydroxy-isopropylamin-Derivate :

(1) 1,3-Dihydroxy-2-methylamino-propan,
(2) 1,3-Dihydroxy-2-äthylamino-propan,
(3) 1,3-Dihydroxy-2-(2'-hydroxyäthyl)-amino-propan,
(4) 1,3-Dihydroxy-2-(2',3'-dihydroxypropyl)-amino-propan,
(5) 1,3-Dihydroxy-2-(2',3',4'-trihydroxy-butyl)-amino-propan,
(6) 1,3-Dihydroxy-2-[N-methyl-N-(2',3',4',5'-tetrahydroxy-pentyl]-amino-propan,
(7) 1,3-Dihydroxy-2-[N-methyl-N-(2',3',4',5',6'-pentahydroxy-hexyl)]-amino-propan,
(8) N,N-Bis-(1,3-dihydroxy-isopropyl)-amin
(9) 1,3-Dihydroxy-2-[tris-(hydroxymethyl)-methyl]-amino-propan,
(10) 1,3-Dihydroxy-2-dimethylamino-propan,
(11) 1,3-Dihydroxy-2-diäthylamino-propan,
(12) 1,3-Dihydroxy-2-[N,N-bis-(2-hydroxyäthyl)]-amino-propan,
(13) 1,3-Dihydroxy-2-[N,N-bis-(2,3-dihydroxypropyl)]-amino-propan,
(14) 1,3-Dihydroxy-2-[N,N-bis-(1,3-dihydroxyisopropyl)]-amino-propan,
(15) 1(N)-(1,3-Dihydroxyisopropyl)-pyrrolidin,
(16) 1(N)-(1,3-Dihydroxyisopropyl)-piperidin,

(17) 4(N)-(1,3-Dihydroxyisopropyl)-morpholin,
(18) 1(N)-(1,3-Hydroxyisopropyl)-piperazin.
Wie erwähnt sind davon als bekannt ermittelt worden die Verbindungen (3), (10), (11), (16) und (17).

## Beispiele

### Beispiel 1

Serinol ( = 1,3-Dihydroxy-isopropylamin)

A. 1,3-Dimethoxy-isopropanol

5,7 kg Natriumhydroxyd werden in 65 kg siedendem Methanol gelöst. Die siedende Lösung wird unter Rühren innert 1 Stunde mit 12,5 kg Epichlorhydrin versetzt, wobei die frei werdende Reaktionswärme die Reaktionsmischung am Sieden erhält. Man kocht noch weiter 6 Stunden am Rückflusskühler. Nun kühlt man auf Raumtemperatur, stellt durch Zusatz von konzentrierter Salzsäure auf pH 7, filtriert das ausgeschiedene Kochsalz ab und unterwirft das Filtrat einer fraktionierten Destillation im Vakuum. Man erhält dabei 14,77 kg 1,3-Dimethoxy-isopropanol vom Siedebereich 63-72 °C/14 Torr., d.s. 91 % der Theorie.

B. 1,3-Dimethoxy-isopropylchlorid

Eine Mischung aus 12 kg 1,3-Dimethoxy-isopropanol, 50 kg Chloroform und 160 g Pyridin wird bei 55-60 °C vorsichtig mit 14,3 kg Thionylchlorid versetzt. Die Reaktionslösung wird während 20 Stunden am Rückflusskühler gekocht, bis die Gasentwicklung (HCl und $SO_2$) vollständig abgeklungen ist.

Nun wird die Reaktionslösung fraktioniert destilliert. Man erhält 12,47 kg (ca. 90 % der Theorie) 1,3-Dimethoxy-isopropylchlorid mit einem Siedebereich von 50-55 °C/Torr.

C. 1,3-Dimethoxy-isopropylamin

1,3 kg 1,3-Dimethoxy-isopropylchlorid werden in 12,8 kg 25 %igem wässerigem Ammoniak während 2 Stunden im Autoklaven auf 170 °C erhitzt. Der überschüssige Ammoniak wird weitgehend abgedampft. Die wässerige Lösung wird mit festem Natriumhydroxyd versetzt. Das 1,3-Dimethoxyisopropylamin wird mit Methylenchlorid extrahiert.

Das Extrakt wird vom Lösungsmittel befreit und im Vakuum destilliert. Man erhält 894,3 g 1,3-Dimethoxyisopropylamin vom Siedebereich 47-49 °C/14 Torr. Kp 760 Torr. 145 °C. Ausbeute : 80 % der Theorie. Diese Verbindung ist mit Wasser und organischen Lösungsmitteln mischbar.

D. 1,3-Dihydroxy-isopropylamin ( = Serinol)

In 3,57 kg 1,3-Dimethoxy-isopropylamin (30 Mol) werden 6,6 kg wässerige 35 %ige Salzsäure einfliessen gelassen. Es entsteht eine Lösung von 1,3-Dimethoxy-isopropylamin·Hydrochlorid in etwa 20-21 %iger Salzsäure. Diese Reaktionslösung wird am Rückflusskühler zum Sieden erhitzt (Badtemperatur ca. 150 °C). Die Ätherspaltung kommt in Gang, es bildet sich unter HCl-Verbrauch Methylchlorid, welches durch den Kühler entweicht. Der HCl-Verlust wird durch Einleiten von HCl-Gas ständig ausgeglichen, derart, dass am Kopf des Rückflusskühlers stets ein minimaler HCl-Gasstrom eben nachweisbar ist, d.h. die Konzentration der siedenden Salzsäure stets etwa im azeotropen Bereich bleibt. Dadurch schreitet die Ätherspaltung rasch voran und ist nach 30 Stunden rückflusskochen bereits vollständig. Die Reaktionslösung wird nun im Vakuum eingedampft. Der ölige Rückstand wird entweder zu Serinol·Hydrochlorid oder zu freier Serinol-Base aufgearbeitet.

Serinol·Hydrochlorid

Der obige ölige Rückstand wird in 5 l Wasser aufgenommen, mit etwas Aktivkohle versetzt, filtriert und im Vakuum vollständig eingedampft. Der Rückstand wird in 4 l siedendem 96 %igem Äthanol gelöst. Beim Abkühlen und Impfen mit Kristallen von 1,3-Dihydroxy-isopropylamin·Hydrochlorid, die durch Reiben einer Probe mit dem Glasstab erzeugt wurden, tritt bei Eistemperatur im Laufe von 2 Stunden eine ziemlich vollständige Kristallisation von Serinol·Hydrochlorid ein. Das Produkt wird abfiltriert und getrocknet.

Ausbeute : 3,45 kg Serinol·Hydrochlorid ( = 1,3-Dihydroisopropylamin·Hydrochlorid), das sind 90 % der Theorie.

Schmelzpunkt : 104-105 °C

Gehalt : 99,8 % (durch Titrieren mit $AgNO_3$ bestimmt).

Dünnschichtchromatogramm (DC) auf Kieselgel mit Laufmittel Chloroform/Methanol/konz. Ammoniak = 6 : 3 : 1. $R_f$ = 0,45.

Serinol-Base

Im öligen Eindampfrückstand, welcher nach der Ätherspaltung erhalten wurde, wird nach Aufnehmen in ca. 10 l Wasser der Chlorid-Gehalt bestimmt. Hierauf wird die dem Chlorid-Gehalt genau äquivalente Menge 30 %ige wässerige Natronlauge (d.s. etwa 4,5 kg) zugesetzt. Das erhaltene Reaktionsgut wird unter Rühren im Vakuum vollständig eingeengt. Das gebildete Kochsalz ist dabei in der geschmolzenen Serinol-Base suspendiert. Der Rückstand wird im Äthanol aufgenommen, das Kochsalz wird abfiltriert und mit Äthanol ausgewaschen. Das Filtrat wird bei Normaldruck eingedampft, wobei der Äthanol zurückgewonnen wird. Der Eindampfrückstand, welcher das Serinol enthält, wird im Feinvakuum destilliert. Man erhält 2,56 kg Serinol (1,3-Dihydroxy-isopropylamin) vom Siedebereich 130-140 °C/0,1 Torr. d.s. 92,6 % d.Th. bezogen auf 1,3-Dimethoxy-isopropylamin.

Schmelzpunkt : 55-56 °C.

D.C. auf Kieselgel mit Laufmittel Chloroform/Methanol/konz. Ammoniak = 6 : 3 : 1. $R_f$ = 0,25.

E. 1,3-Dihydroxy-isopropylamin durch Hydrolyse von 1,3-Dimethoxy-isopropylamin mit Bromwasserstoffsäure

59,5 g 1,3-Dimethoxy-isopropylamin werden in 950 g 47 %ige wässerige Bromwasserstoffsäure eingetragen. Die Rekationslösung wird während 5 Stunden am Rückflusskühler gekocht und anschliessend zur Trockene eingedampft. Der Rückstand wird in Wasser gelöst, die Lösung wird mit Aktivkohle entfärbt und durch eine mit stark saurem Kationenaustauscherharz (z.B. 500 ml Amberlite(R) IR-120) beschickte Säule perkolieren gelassen. Die ausfliessende $Br^{(-)}$-haltige Lösung wird verworfen. Das Serinol wird mit 2N Ammoniak vom Ionenaustauscher abgelöst. Die basische Lösung wird eingedampft und der Rückstand im Vakuum destilliert.

Man erhält 38,7 g Serinol vom Siedebereich 117-122 °C/0,05 Torr. d.s. 85 % d.Th.

DC mit Laufmittel Dioxan/konz. Ammoniak/Wasser = 6 : 1 : 2. $R_f$ = 0,55.

## Beispiel 2

1,3-Dihydroxy-2-methylamino-propan

A. 1,3-Dimethoxy-2-methylamino-propan

222 g 1,3-Dimethoxy-isopropylchlorid werden in 1 000 g 40 %iger wässeriger Methylaminlösung im Autoklaven während 2 Stunden auf 170 °C erhitzt. Die Reaktionslösung wird eingedampft, der Rückstand wird mit ca. 80 g festem Natriumhydroxyd versetzt. Die sich ausscheidende organische Phase wird mit Diäthyläther extrahiert. Das Extrakt wird vom Lösungsmittel befreit und anschliessend im Vakuum destilliert.

Man erhält 179 g 1,3-Dimethoxy-2-methylamino-propan, d.s. 84 % der Theorie, vom Siedepunkt 48 °C/11-12 Torr.

Äquivalentgewicht : ber. 133,19 ; gef. 133,71 (titr. mit 0,1N $HClO_4$).

Dünnschichtchromatogramm (DC) auf Kieselgel mit Laufmittel Chloroform/Methanol/Ammoniak (15 %ig) = 85 : 14 : 1, 1 Flecken bei $R_f$ = 0,70.

B. 1,3-Dihydroxy-2-methylamino-propan

66,6 g 1,3-Dimethoxy-2-methylamino-propan werden in 1 000 ml 20 %iger Salzsäure während 56 Stunden am Rückflusskühler gekocht, bis aufgrund periodischer Kontrollen mittels Dünnschichtchromatographie nachgewiesen werden kann, dass alles Ausgangsmaterial verschwunden ist und sich ein weitgehend einheitliches Produkt gebildet hat. Die Reaktionslösung wird zur Trockene verdampft. Der Rückstand wird in 100 ml Wasser gelöst und auf ein stark saures Kationenaustauscherharz (z.B. Amberlite(R) IR 120) gegeben. Der Ionenaustauscher wird mit Wasser $Cl^{(-)}$-frei gewaschen. Das Produkt wird nun mit 2 000 ml 2N Ammoniak aus dem Harz eluiert. Das Eluat wird eingedampft. Der Rückstand wird im Vakuum fraktioniert destilliert.

Man erhält 2 Fraktionen :

1. Kp 80-81 °C 1 Torr. Äquivalentgewicht gef. 120
2. Kp 80-82 °C/0,1 Torr. Äquivalentgewicht gef. 105,7.

Die 1. Fraktion von 3,9 g besteht aus 1-Methoxy-3-hydroxy-2-methylamino-propan (Äquivalentgewicht ber. 119,17), d.s. 6,5 % der Theorie.

Die 2. Fraktion von 42 g besteht aus 1,3-Dihydroxy-2-methylamino-propan (Äquivalentgewicht ber. 105,1), d.s. 80 % der Theorie.

DC auf Kieselgel mit Laufmittel Methylenchlorid/Methanol/konz. Ammoniak = 6 : 3 : 1.

1,3-Dihydroxy-2-methylamino-propan $R_f$ = 0,46.

1-Methoxy-3-hydroxy-2-methylaminopropan $R_f$ = 0,91.

Das Pikrat von 1,3-Dihydroxy-2-methylamino-propan schmilzt nach Umkristallisieren aus Äthanol bei 104-105 °C.

## Beispiel 3

1,3-Dihydroxy-2-äthylamino-propan

A. 1,3-Diäthoxy-isopropylchlorid

Diese Verbindung wird analog Beispiel 1A./B. erhalten durch Umsetzung von Epichlorhydrin mit Äthanol in Gegenwart von Kaliumhydroxyd zum 1,3-Diäthoxy-isopropanol (Siedepunkt 190 °C/760 Torr., bzw. 85 °C/12 Torr.) und Behandlung desselben mit Thionylchlorid in der Siedehitze und in Gegenwart von wenig Pyridin.

Siedepunkt : 62 °C/14 Torr.

B. 1,3-Diäthoxy-2-äthylamino-propan

83,4 g 1,3-Diäthoxy-isopropylchlorid werden mit 200 ml 70 %igem wässrigem Äthylamin im Autoklaven während 2-4 Stunden auf 175 °C erhitzt. Die Reaktionsmischung wird wie im Beispiel 2 A.

beschrieben aufgearbeitet. Man erhält 70 g 1,3-Diäthoxy-2-äthylamino-propan vom Siedepunkt 65 °C/14 Torr., das sind 80 % der Theorie.

C. 1,3-Dihydroxy-2-äthylamino-propan

52,6 g 1,3-Diäthoxy-2-äthylamino-propan werden in 900 g 47 %ige Bromwasserstoffsäure eingetragen. Die Reaktionslösung wird 7-9 Stunden am Rückfluss gekocht und anschliessend analog Beispiel 1 E. aufgearbeitet. Man erhält 28,3 g 1,3-Dihydroxy-2-äthylamino-propan vom Siedepunkt 88 °C/0,2 Torr., das sind 79 % der Theorie. Die Verbindung ist leicht löslich in Wasser.

## Beispiel 4

1,3-Dihydroxy-2-dimethylamino-propan

A. 1,3-Dimethoxy-2-dimethylamino-propan

111 g 1,3-Dimethoxy-isopropylchlorid werden in 350 g 40 %igem wässerigem Dimethylamin im Autoklaven während 2-4 Stunden auf 170 °C erhitzt.

Die Aufarbeitung erfolgt nach der im Beispiel 2 A. beschriebenen Methode. Man erhält 1,3-Dimethoxy-2-dimethylamino-propan vom Siedebereich 50-53 °C/12-14 Torr., das sind 85 % der Theorie.

B. 1,3-Dihydroxy-2-dimethylamino-propan

100 g 1,3-Dimethoxy-2-dimethylamino-propan werden in 1 000 ml 20 %iger Salzsäure während 70 Stunden am Rückfluss gekocht und danach analog Beispiel 2 aufgearbeitet. Man erhält 69,64 g 1,3-Dihydroxy-2-dimethylamino-propan vom Siedepunkt 90-92 °C/0,6 Torr., das sind 86 % der Theorie.

## Beispiel 5

1,3-Dihydroxy-2-diäthylamino-propan

Diese Verbindung wird in analoger Weise durch Erhitzen von 69,3 g 1,3-Dimethoxy-isopropylchlorid mit 110 g Diäthylamin im Autoklaven und Hydrolyse des erhaltenen 1,3-Dimethoxy-2-diäthylamino-propans (Kp : 65 °C/16 Torr.) durch Kochen mit azeotroper wässriger Salzsäure erhalten.

Siedepunkt : 115 °C/0,4 Torr. Die Verbindung ist sehr gut löslich in Wasser.

## Beispiel 6

1,3-Dihydroxy-2-(2'-hydroxyäthyl)-amino-propan

A. 1,3-Dimethoxy-2-(2'-hydroxyäthyl)-amino-propan

138,6 g 1,3-Dimethoxy-isopropylchlorid (1 Mol) werden mit 250 g 2-Aminoäthanol vermischt und einige Stunden auf 160-165 °C erhitzt. Das nicht umgesetzte überschüssige 2-Aminoäthanol wird im Vakuum über eine Kolonne abdestilliert. Der Eindampfrückstand wird mit festem Natriumhydroxyd versetzt und das Produkt mit Diäthyläther wiederholt extrahiert. Das Extrakt wird eingedampft und der Rückstand im Vakuum destilliert. Man erhält 148,5 g 1,3-Dimethoxy-2-(2'-hydroxyäthyl)-amino-propan, d.s. 91 % der Theorie, vom Siedepunkt 124-126 °C/11 Torr.

Äquivalentgewicht : ber. 163,2 ; gef. 163,9 (titr. mit 0,1N $HClO_4$).

DC auf Kieselgel mit Laufmittel Chloroform/Methanol/Ammoniak (25 %ig) = 85 : 14 : 1.

1 Flecken, $R_f = 0{,}45$.

B. 1,3-Dihydroxy-2-(2'-hydroxyäthyl)-amino-propan

81,6 g 1,3-Dimethoxy-2-(2'-hydroxyäthyl)-amino-propan werden in 1 000 ml 20 %iger Salzsäure während 65 Stunden am Rückfluss gekocht bis im DC die Flecken des Ausgangsmaterials verschwunden sind und ein einheitliches neues Produkt entstanden ist. Die Aufarbeitung erfolgt wie in den Beispielen 1 D. oder 2 B. beschrieben. Man erhält 59,5 g 1,3-Dihydroxy-2-(2'-hydroxyäthyl)-amino-propan vom Siedebereich 138-140 °C/0,05 Torr., d.s. 88 % der Theorie.

Äquivalentgewicht : ber. 135,17 ; gef. 135,86.

DC auf Kieselgel mit Laufmittel Methylenchlorid/Methanol/konz. Ammoniak = 6 : 3 : 1 ; $R_f = 0{,}49$.

Das Pikrat von 1,3-Dihydroxy-2-(2'-hydroxyäthyl)-aminopropan schmilzt bei 103-104 °C.

## Beispiel 7

1(N)-(1,3-Dihydroxyisopropyl)-piperazin

A. 1(N)-(1,3-Dimethoxy-isopropyl)-piperazin

138,6 g 1,3-Dimethoxy-isopropylchlorid werden mit 430 g Piperazin vermischt, geschmolzen und unter Rühren während 4-6 Stunden auf 140-145 °C gerührt.

Nach dem Abkühlen werden die Phasen getrennt. Die leichtere Phase wird durch Destillation im Vakuum vom überschüssigen Piperazin befreit. Der Destillationsrückstand wird filtriert und mit Diäthyläther gewaschen. Das Filtrat wird eingedampft und der Rückstand im Vakuum abdestilliert.

Man erhält 152,5 g 1(N)-(1,3-Dimethoxy-isopropyl)-piperazin, d.s. 81 % der Theorie ; vom Siedepunkt 121-124 °C/11 Torr.

Äquivalentgewicht : ber. 94,14 ; gef. 93,75.

DC auf Kieselgel mit Laufmittel Isobutanol/Isopropanol/Ammoniak (25 %ig) = 35 : 35 : 30. 1 Flecken $R_f = 0,67$.

B. 1(N)-(1,3-Dihydroxyisopropyl)-piperazin

65,9 g 1(N)-(1,3-Dimethoxy-isopropyl)-piperazin werden in 740 ml 20 %iger Salzsäure 65 Stunden am Rückflusskühler gekocht. Die Aufarbeitung erfolgt analog wie in den Beispielen 1 D. oder 2 B. beschrieben.

Man erhält beim Destillieren 2 Fraktionen :

1. Kp 100 °C/0,5 Torr. Äquivalentgewicht : 87,72 ; Menge : 6,1 g

2. Kp 125 °C/0,05 Torr. Äquivalentgewicht : 80,31 ; Menge : 41 g

Beide Produkte kristallisieren beim Stehen-lassen.

Die 1. Fraktion besteht aus 1(N)-(1-Methoxy-3-hydroxy-isopropyl)-piperazin (Äquivalentgewicht ber. 87,13), Ausbeute : 10 % ; Fp 50 °C.

Die 2. Fraktion besteht aus 1(N)-(1,3-Dihydroxy-isopropyl)-piperazin (Äquivalentgewicht ber. 80,11) ; Ausbeute 73 % der Theorie.

Schmelzpunkt (nach Umkristallisation aus Isopropanol) : 113-115 °C.

DC auf Kieselgel mit Laufmittel Methylenchlorid/Methanol/konz. Ammoniak : 6 : 3 : 1.

$R_f$ (1. Fraktion) : 0,79

$R_f$ (2. Fraktion) : 0,45.

Beispiel 8

4(N)-(1,3-Dihydroxy-isopropyl)-morpholin

A. 4(N)-(1,3-Dimethoxy-isopropyl)-morpholin

69,3 g 1,3-Dimethoxy-isopropylchlorid (0,5 Mol) werden mit 87 g (1 Mol) Morpholin vermischt und einige Stunden auf ca. 160-165 °C erhitzt. Das Produkt wird durch Extrahieren des Reaktionsgutes mit Isopropanol vom Morpholin·Hydrochlorid getrennt und anschliessend im Vakuum destilliert. Ausbeute : 76 g, d.s. 80 % der Theorie.

B. 4(N)-(1,3-Dihydroxy-isopropyl)-morpholin

75,7 g 4(N)-(1,3-Dimethoxy-isopropyl)-morpholin werden in 900 ml 20 %iger Salzsäure etwa 70 Stunden am Rückfluss gekocht und anschliessend etwa analog Beispiel 7 aufgearbeitet.

Man erhält 48,4 g 4(N)-(1,3-Dimethoxy-isopropyl)-morpholin vom Siedepunkt 120 °C/0,15 Torr. Ausbeute 75 % der Theorie.

Beispiel 9

1(N)-(1,3-Dihydroxy-isopropyl)-piperidin

A. 1(N)-(1,3-Dimethoxy-isopropyl)-piperidin

69,3 g 1,3-Dimethoxy-isopropylchlorid (0,5 Mol) werden mit 85 g Piperidin (1 Mol) vermischt, im Bombenrohr einige Stunden auf 170 °C erhitzt und anschliessend wie im Beispiel 8 beschrieben aufgearbeitet.

Ausbeute : 71,14 g, das sind 76 % der Theorie.

B. 1(N)-(1,3-Dihydroxy-isopropyl)-piperidin

70,2 g 1(N)-(1,3-Dimethoxy-isopropyl)-piperidin (0,375 Mol) werden in 850 ml 20 %iger Salzsäure 70 Stunden am Rückfluss gekocht und anschliessend ähnlich wie in den vorstehenden Beispielen beschrieben aufgearbeitet.

Man erhält 50,75 g 1(N)-(1,3-Dihydroxy-isopropyl)-piperidin vom Siedepunkt 125 °C/1-2 Torr., das sind 85 % der Theorie.

Beispiel 10

1(N)-(1,3-Dihydroxy-isopropyl)-pyrrolidin

Diese Verbindung wird in analoger Weise durch Erhitzen von 69,3 g 1,3-Dimethoxy-isopropylchlorid mit 71,1 g Pyrrolidin im Bomberrohr und Hydrolyse des erhaltenen 1(N)-(1,3-Dimethoxy-isopropyl)-pyrrolidin durch Kochen mit wässriger 20 %iger Salzsäure erhalten.

Siedepunkt : 120 °C/2-4 Torr. Die Verbindung ist wasserlöslich.

Beispiel 11

1,3-Dihydroxy-2-(2',3'-dihydroxypropyl)-amino-propan

A. 1,3-Dimethoxy-2-(2',3'-dihydroxypropyl)-amino-propan

138,6 g 1,3-Dimethoxy-isopropylchlorid (1 Mol) werden mit 182,2 g 2,3-Dihydroxypropylamin (2 Mol) einige Stunden auf 160-165 °C erhitzt.

Der Rückstand wird zwischen Chloroform und Wasser verteilt. Die Chloroform-Lösung wird verdampft. Der Eindampfrückstand besteht aus 170 g 1,3-Dimethoxy-2-(2',3'-dihydroxypropyl)-amino-propan, das sind 88 % der Theorie.

B. 1,3-Dihydroxy-2-(2',3'-dihydroxypropyl)-amino-propan

96,6 g 1,3-Dimethoxy-2-(2',3'-dihydroxypropyl)-amino-propan (0,5 Mol) werden mit 1 000 ml 20 %iger Salzsäure während 65 Stunden am Rückfluss gekocht und anschliessend nach der im Beispiel 2 B. beschriebenen Methode aufgearbeitet. Man erhält 64,4 g leicht wasserlösliches 1,3-Dihydroxy-2-(2',3'-dihydroxypropyl)-amino-propan, welches bei ca. 155 °C/0,05 Torr. siedet.

Ausbeute 78 % der Theorie.

## Beispiel 12

N,N-Bis-(1,3-dihydroxy-isopropyl)-amin

Diese Verbindung wird analog Beispiel 11 durch Erhitzen von 1,3-Dimethoxy-isopropylchlorid (69,3 g) mit Serinol (91,1 g) und Hydrolyse des dabei erhaltenen 1,3-Dimethoxy-2-(1',3'-dihydroxyisopropyl)-amino-propan's (82 g) durch Kochen mit 20 %iger Salzsäure (1 000 ml) gewonnen. N,N-Bis-(1,3-dihydroxy-isopropyl)-amin siedet bei ca. 150 °C/0,05 Torr. und ist spielend leicht wasserlöslich.

## Beispiel 13

1,3-Dihydroxy-2-[N-methyl-N-(2',3',4',5'-tetrahydroxy-pentyl)]-amino-propan

Beim Erhitzen von 69,3 g (0,5 Mol) 1,3-Dimethoxy-isopropylchlorid mit 165,2 g (1,0 Mol) N-Methyl-D-xylamin ( = 1-Methylamino-1-desoxy-D-xylit [Dorn et al, Chem. Ber. *99* 812 (1966)]) während einiger Stunden auf 160-170 °C erhält man 1,3-Dimethoxy-2-[N-methyl-N-(2',3',4',5'-tetrahydroxypentyl)]-amino-propan.

Dieser Äther wird analog Beispiel 6 und 11 durch Rückflusskochen mit 20 %iger Salzsäure zu 1,3-Dihydroxy-2-[N-methyl-N-(2',3',4',5'-tetrahydroxy-pentyl)]-amino-propan hydrolysiert. Die neue Verbindung bildet zerfliessende farblose Kristalle, die an der Luft begierig $CO_2$ aufnehmen und sich dabei teilweise ins Carbonat umwandeln. Die Verbindung ist spielend leicht wasserlöslich.

## Beispiel 14

1,3-Dihydroxy-2-[N-methyl-N-(2',3',4',5',6'-pentahydroxyhexyl)]-amino-propan

69,3 g 1,3-Dimethoxy-isopropylchlorid (0,5 Mol) und 195,3 g N-Methyl-D-glucamin [ = 1-Deoxy-1-methylamino-D-glucitol] (1 Mol) werden während 3 Stunden auf 170 °C erhitzt. Durch Auskochen mit Äthanol oder Isopropanol kann das gewünschte Zwischenprodukt vom schwerer löslichen, bei der Umsetzung entstandenen N-Methyl-D-glucamin·Hydrochlorid abgetrennt werden.

1,3-Dimethoxy-2-[N-methyl-N-(2',3',4',5',6'-pentahydroxy-hexyl)]-amino-propan wird durch Rückflusskochen mit 20 %iger Salzsäure zu 1,3-Dihydroxy-2-[N-methyl-N-(2',3',4',5',6'-pentahydroxy-hexyl)]-amino-propan gespalten.

Diese neue Verbindung ist eine starke, leicht wasserlösliche Base, die aus der Luft $CO_2$ bindet und dabei teilweise in das Carbonat umgewandelt wird. Sie bildet farblose Kristalle, die bei ca. 130 °C schmelzen.

## Ansprüche

1. Verfahren zur Herstellung von Serinol und von am Stickstoffatom substituierten Serinol-Derivaten, dadurch gekennzeichnet, dass man ein aus Epichlorhydrin über 1,3-Dialkoxy-isopropanol nach bekannten Methoden leicht erhältliches niedriges 1,3-Dialkoxy-isopropylhalogenid mit Ammoniak oder einem Amin der allgemeinen Formel (I)

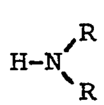

(I)

worin R vorzugsweise Wasserstoff, Methyl, Äthyl, Hydroxyäthyl, Dihydroxypropyl, oder zusammen mit R' und dem N-Atom Bestandteil eines Pyrrolidin-, Piperidin-, Morpholin- oder Piperazin-Ringes und R' Wasserstoff, Methyl, Äthyl oder Hydroxyalkyl, Di-, Tri-, Tetra- oder Pentahydroxyalkyl mit 2 bis 6 C-Atomen oder zusammen mit R und dem N-Atom einen der genannten heterocyklischen Ringe bedeuten, umsetzt, und im erhaltenen 1,3-Dialkoxy-isopropylamin bzw. 1,3-Dialkoxy-isopropyl-amin-Derivat der Formel (II)

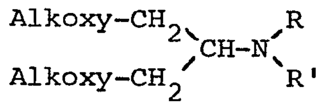 (II)

worin Alkoxy niedrige Alkoxy-Reste mit 1 bis 3 Kohlenstoff-Atomen sind und R sowie R′ die oben definierte Bedeutung haben, die Äthergruppen durch Erhitzen mit einer Halogenwasserstoffsäure spaltet.

2. Bevorzugtes Verfahren nach Patentanspruch 1, dadurch gekennzeichnet, dass man dazu 1,3-Dimethoxyisopropylchlorid als Zwischenprodukt verwendet und die Ätherspaltung durch Erhitzen mit Salzsäure durchführt.

3. Technisches Verfahren zur Herstellung von Serinol nach Patentansprüchen 1 und 2, dadurch gekennzeichnet, dass man 1,3-Dimethoxy-isopropylchlorid zunächst mit überschüssigem Ammoniak umsetzt und erhaltenes 1,3-Dimethoxy-isopropylamin durch Rückflusskochen in wässriger Salzsäure in Serinol überführt, wobei man zur Beschleunigung der Reakfion verbrauchten Chlorwasserstoff laufend oder periodisch ersetzt.

**Claims**

1. Process for the preparation of serinol, and of serinol derivatives substituted at the nitrogen atom, characterised in that a lower 1,3-dialkoxy-isopropyl halide, which is easily obtainable from epichlorohydrin via 1,3-dialkoxy-isopropanol in accordance with known methods, is reacted with ammonia or an amine of the general formula (I)

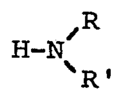 (I)

wherein R preferably denotes hydrogen, methyl, ethyl, hydroxyethyl or dihydroxypropyl or together with R′ and the N-atom denotes part of a pyrrolidine, piperidine, morpholine or piperazine ring and R′ denotes hydrogen, methyl, ethyl, hydroxyalkyl, dihydroxyalkyl, trihydroxyalkyl, tetrahydroxyalkyl or pentahydroxyalkyl having 2 to 6 C atoms or together with R and the N atom denotes one of the heterocyclic rings mentioned, and that, in the resulting 1,3-dialkoxy-isopropylamine or 1,3-dialkoxy-isopropylamine derivative of the formula (II)

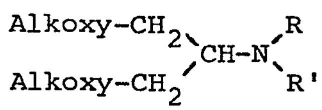 (II)

wherein Alkoxy are lower alkoxy radicals having 1 to 3 carbon atoms and R and R′ have the meaning defined above, the ether groups are cleaved by heating with a hydrogen halide acid.

2. Preferred process according to Patent Claim 1, characterised in that 1,3-dimethoxyisopropyl chloride is used as the intermediate product and the ether cleavage is effected by heating with hydrochloric acid.

3. Industrial process for the preparation of serinol according to Patent Claims 1 and 2, characterised in that 1,3-dimethoxy-isopropyl chloride is first reacted with excess ammonia, and the 1,3-dimethoxy-isopropylamine obtained is converted to serinol by boiling in aqueous hydrochloric acid under reflux, consumed hydrogen chloride being replaced continuously or periodically in order to accelerate the reaction.

**Revendications**

1. Procédé de préparation du sérinol et de dérivés du sérinol substitué sur l'atome d'azote, caractérisé en ce que l'on fait réagir un halogénure de 1,3-dialcoxy-isopropyle inférieur lui-même facilement obtenu selon des procédés connus à partir de l'épichlorhydrine en passant par l'intermédiaire d'un 1,3-dialcoxy-isopropanol, avec l'ammoniac ou une amine de formule générale I

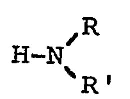 (I)

dans laquelle R représente de préférence l'hydrogène, un groupe méthyle, éthyle, hydroxyéthyle, dihydroxypropyle ou forme avec R' et l'atome d'azote une partie d'un noyau pyrrolidine, pipéridine, morpholine ou pipérazine, et R' représente l'hydrogène, un groupe méthyle, éthyle, ou hydroxyalkyle, di-, tri-, tétra- ou penta-hydroxyalkyle en C2-C6, ou forme avec R et l'atome d'azote l'un des noyaux hétérocycliques mentionnés, et dans la 1,3-dialcoxy-isopropylamine ou le dérivé de 1,3-dialcoxy-isopropylamine respectivement obtenu, qui répond à la formule II

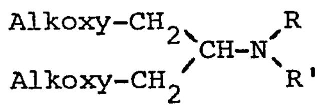

(II)

dans laquelle Alkoxy représente des groupes alcoxy inférieurs en C1-C3 et R et R' ont les significations indiquées ci-dessus, on élimine les groupes éthers par chauffage avec un hydracide halogéné.

2. Procédé préféré selon la revendication 1, caractérisé en ce que l'on utilise en tant que produit intermédiaire le chlorure de 1,3-diméthoxy-isopropyle et on élimine les groupes éthers par chauffage avec l'acide chlorhydrique.

3. Procédé industriel pour la préparation du sérinol selon les revendications 1 et 2, caractérisé en ce que l'on fait d'abord réagir le chlorure de 1,3-diméthoxy-isopropyle avec l'ammoniac en excès et on convertit la 1,3-diméthoxy-isopropylamine obtenue, par ébullition ou au reflux dans l'acide chlorhydrique aqueux, en sérinol, en remplaçant en continu ou périodiquement le chlorure d'hydrogène consommé pour accélérer la réaction.